# EUROPEAN PATENT SPECIFICATION

(11) **EP 4 564 000 B1**
(45) Date of publication and mention of the grant of the patent: **02.09.2026**
(21) Application number: 24208045.5
(22) Date of filing: 22.10.2024
(51) Int. Cl.: G01N 33/28

(54) **MAGNETIC OIL DEBRIS SENSOR**
MAGNETISCHER ÖLABRIEBSENSOR
CAPTEUR MAGNÉTIQUE DE DÉBRIS D'HUILE

(30) Priority: 30.11.2023 IN 202311081383
(43) Date of publication of application: 04.06.2025
(73) Proprietor: Honeywell International Inc., Charlotte, NC 28202 (US)
(72) Inventor: S, Prathap, Charlotte, 28202 (US); SAJJAN, Murgesh, Charlotte, 28202 (US); M N, Nithin, Charlotte, 28202 (US); JERRED, John P, Charlotte, 28202 (US); WILLIS, Christopher, Charlotte, 28202 (US)
(74) Representative: Haseltine Lake Kempner LLP

(56) References cited:
- CN-A- 117 074 511
- DE-A1- 102009 020 582
- US-A- 5 321 355

## Description

### FIELD OF THE INVENTION

Example embodiments of the present disclosure relate generally to magnetic oil plugs, and in particular, integrating a Hall-effect based ferrous debris monitoring sensor into a magnetic oil plug.

### BACKGROUND

Monitoring system fluids (e.g., engine, transmission, gearbox, radiators/cooling, etc.) for metal debris caused by system component (e.g., bearings, gears, etc.) wear and/or failure may be essential for determining the health of a system. For example, monitoring an amount of metal debris in system fluids may be used to determine whether preventative maintenance should be performed to avoid costly damage to system/components and/or dealing with unscheduled failures leading to safety concerns, downtime/lost time cost, lost production, and unnecessary overtime. Monitoring an amount of metal debris in system fluids may also allow for avoiding prematurely performing maintenance on or replacing equipment when not needed.

Conventional approaches for determining metal debris in system fluids may comprise using passive, nonelectric, magnetic debris plugs. For example, a conventional oil debris plugs may accumulate ferrous debris from system components (bearings, gears, etc.) wear and/or failure. However, conventional oil debris plugs may only be examined during routine maintenance

DE 10 2009 020582 A1 discloses a debris sensor comprising a Hall effect sensor and a combination of a permanent magnet, a pole piece and a magnetic coil. The magnetic field strength at a collection point may be adjusted by varying the current through the magnetic coil, thereby moving the pole piece.

Applicant has identified shortcomings associated with conventional debris plugs.

### BRIEF SUMMARY

Various embodiments described herein relate to components, apparatuses, and systems for detecting ferrous debris deposition on a debris sensor apparatus.

The present invention is defined by the independent claim, to which reference should now be made. Specific embodiments are defined in the dependent claims. In accordance with various embodiments of the present disclosure, a debris sensor apparatus according to claim 1 is provided.

In some embodiments, the debris sensor apparatus further comprises a printed circuit board assembly coupled to the Hall-effect sensor. In some embodiments, the printed circuit board comprises one or more processors and a communication interface configured to transmit signal output associated with the Hall-effect sensor to a data analysis computing entity. In some embodiments, the signal output is representative of debris measurement data comprising one or more values of magnetic flux density. In some embodiments, the data analysis computing entity is configured to generate one or more actions responsive to the debris measurement data exceeding a debris threshold. In some embodiments, the debris threshold is associated with a maximum rate of change associated with debris deposition on the shaft or a maximum amount of debris deposition on the shaft.

In some embodiments, the Hall-effect sensor is configured to detect magnetic flux density. In some embodiments, the Hall-effect sensor is configured to detect an increase to the magnetic flux density in proportion to an amount of ferrous debris deposition on the shaft. In some embodiments, the one or more permanent magnets comprise an array of a plurality of magnet elements and one or more non-magnetic spacers configured therebetween the plurality of magnet elements. In some embodiments, the Hall-effect sensor is configured along a central axis of the one or more permanent magnets. In some embodiments, the top piece and the bottom pole piece comprise flux concentrators that concentrate magnetic flux associated with the one or more permanent magnets through the Hall-effect sensor and through a length of a periphery of shaft 106. In some embodiments, the debris sensor assembly is encapsulated and/or sealed within the shaft. In some embodiments, the shaft comprises a non-ferrous housing. In some embodiments, the Hall-effect sensor is oriented in the cavity at a given angle with respect to an axis of the one or more permanent magnets. In some embodiments, the second pole piece comprises a funnel shape towards the Hall-effect sensor. In some embodiments, the debris sensor assembly further comprises a gap between the bottom pole piece and the Hall-effect sensor.

The foregoing illustrative summary, as well as other exemplary objectives and/or advantages of the disclosure, and the manner in which the same are accomplished, are further explained in the following detailed description and its accompanying drawings.

### BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1A illustrates a debris sensor apparatus in accordance with various embodiments of the present disclosure;
FIG. 1B illustrates a debris sensor apparatus in accordance with other various embodiments of the present disclosure;
FIG. 2 illustrates a cross-sectional view of a debris sensor apparatus in accordance with various embodiments of the present disclosure;
FIG. 3 illustrates magnetic flux characteristics of an example debris sensor apparatus in accordance with various embodiments of the present disclosure;
FIG. 4 illustrates an example relationship between magnetic flux density and debris deposition on a debris sensor apparatus in accordance with various embodiments of the present disclosure.
FIG. 5 illustrates an example overview of an architecture in accordance with various embodiments of the present disclosure.
FIG. 6 illustrates an example data analysis computing entity in accordance with various embodiments of the present disclosure.
FIG. 7 illustrates an example method for monitoring debris deposition in an operating environment.

### DETAILED DESCRIPTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

As used herein, terms such as "front," "rear," "top," etc., are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," and the like generally mean that the particular feature, structure, or characteristic following the phrase may be included in at least one embodiment of the present disclosure, and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments, or it may be excluded.

As described above, although magnetic debris plugs may accumulate debris indicating system component wear and/or failure, the magnetic debris plugs may only be examined during routine maintenance. Thus, there is a need for real-time monitoring of debris accumulated on a magnetic debris plug.

Various example embodiments of the present disclosure provide various technical advancements and improvements to magnetic debris plugs. In accordance with various examples of the present disclosure, a debris sensor apparatus is disclosed. A debris sensor apparatus may comprise a sensor configured for real-time monitoring of debris levels accumulated on the debris sensor apparatus when the debris sensor apparatus is configured within an operating environment. As such, a debris sensor apparatus according to various embodiments of the present disclosure may be used to detect debris level in real-time, ondemand, or periodically to ensure that appropriate actions may be taken in time to avoid negative consequences and allow maintenance to be planned on or before scheduled downtime.

The disclosed debris sensor apparatus may be used in a plurality of applications and is not limited to any specific application as disclosed herewith. The plurality of applications may include hydraulic system monitoring, drive train system monitoring, wind turbine condition monitoring, condition monitoring in mining industries, or crane gearbox monitoring. In some example embodiments, the debris sensor apparatus comprises a debris sensor plug. A debris sensor plug may be inserted and secured into a recess of an operating environment, such as a device or machinery, to monitor wear or failure of the operating environment in real-time.

According to various embodiments of the present disclosure, a Hall-effect sensor may be configured with a debris sensor apparatus to generate data readings (analog or digital) representative of a quantity of debris deposited on a shaft of the debris sensor apparatus in conjunction with concentrated magnetization of at least a portion of the shaft for ferrous debris collection. The Hall-effect sensor may be configured to detect collection of debris on the debris sensor apparatus. As such, real-time monitoring may be performed using the debris sensor apparatus to determine actions that may be appropriate for current debris levels to avoid negative consequences and allows maintenance to be planned during scheduled downtime. For example, a debris sensor apparatus may be configured with a computing entity to perform condition-based monitoring that protects critical rotating assets by directly measuring ferrous wear within an equipment. In the event of unpredictable failure, an immediate response may be determined as appropriate such that any further negative effects may be avoided. Data may be received from the Hall-effect sensor and used to generate trending, prediction patterns, and analysis to obtain a variety of information.

In some embodiments, a debris sensor apparatus comprises a head portion, a male-threaded portion, and a shaft. The shaft may comprise a hollow chamber housing in which a debris sensor assembly may be disposed. In some embodiments, a debris sensor assembly comprises one or more permanent magnets (e.g., rare-earth magnets), a Hall-effect sensor, a plurality of pole pieces (or flux concentrators, a power source, and a printed circuit board assembly (PCBA). In some example embodiments, at least a portion of the one or more permanent magnets comprises a cavity in which the Hall-effect sensor may be configured within. The cavity comprises a null field space suitable for inserting a Hall-effect sensor therein to reduce magnetic flux induced on the Hall-effect sensor. For example, a debris sensor apparatus may comprise one or more permanent magnets having a desired magnetic field strength (e.g., sufficient to accumulate ferrous debris) but greater than typical saturation limits of Hall-effect sensors (e.g., +/-160-200mT). As such, a Hall-effect sensor may be configured within a cavity of one or more permanent magnets, as disclosed herewith, such that the Hall-effect sensor may be used with the one or more permanent magnets in applications where the magnetic field strength of the one or more permanent magnets exceeds the saturation limits of the Hall-effect sensor.

In some embodiments, the Hall-effect sensor is mounted on a first side of the PCBA and the first side of the PCBA may be interfaced with a portion of the one or more permanent magnets comprising the cavity such that the Hall-effect sensor is encompassed within the cavity but without physical contact with the one or more permanent magnets. In some embodiments, the one or more permanent magnets interfaced with the PCBA is further configured between a top pole piece and a bottom pole piece. The top pole piece and the bottom pole piece may be configured along the distal and proximal ends of the shaft. In some embodiments, the top pole piece and the bottom pole piece may direct or change magnetic flux direction emanating from the one or more permanent magnets and further prevent saturation of the Hall-effect sensor.

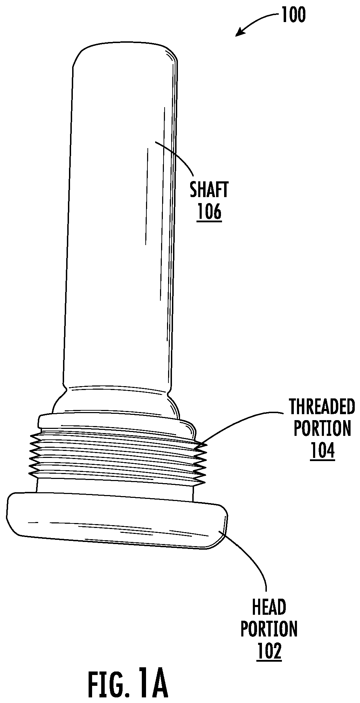

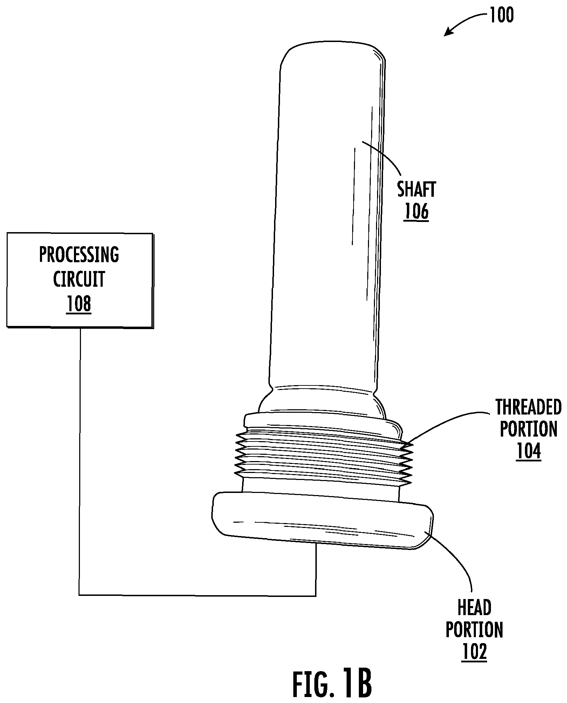

Referring now to FIG. 1A, a debris sensor plug 100 is depicted, which may be used in accordance with various embodiments of the present disclosure. The debris sensor plug 100 comprises a head portion 102, a male-threaded portion 104, and a shaft 106. The head portion 102 may comprise the widest portion of the debris sensor plug 100 that prohibits the debris sensor plug 100 from being driven deeper than the length of the male-threaded portion 104 (e.g., into a female-threaded recess). The head portion 102 may also comprise a bearing surface for allowing the debris sensor plug 100 to turn such that the male-threaded portion 104 may be fastened to a female-threaded recess. As depicted in FIG. 1, components of debris sensor plug 100, such as head portion 102, male-threaded portion 104, and shaft 106 comprise cylindrical shapes, e.g., to fit into a cylindrical recess, however, in other embodiments, any one of the components may comprise other shapes, e.g., to fit into respective other-shaped recesses. In some embodiments, debris sensor plug 100 is coupled to a processing circuit 108, as depicted in FIG. 1B. Processing circuit 108 may comprise a microcontroller, microprocessor, or computing entity and a communication interface for transmitting digital or analog signal output from a Hall-effect sensor embedded within the debris sensor plug 100 to a monitoring computing system.

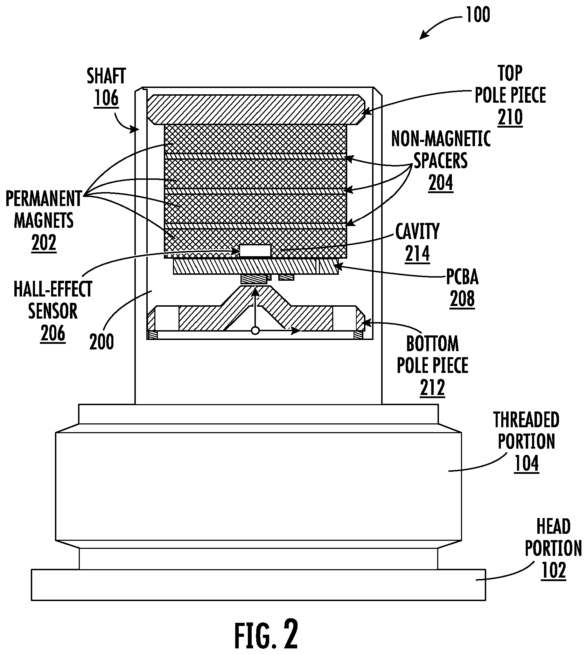

FIG. 2 is a cross-section view of the debris sensor plug 100 in accordance with various embodiments of the present disclosure. As depicted in FIG. 2, shaft 106 comprises an inner chamber in which components of a debris sensor assembly 200 are disposed. Debris sensor assembly 200 comprises one or more permanent magnets 202 and non-magnetic spacers 204. The one or more permanent magnets 202 comprises a plurality of magnet elements that are stacked in an array with the non-magnetic spacers 204 therebetween each of the plurality of magnet elements. The one or more permanent magnets 202 may be axially magnetized such that the magnetic field at the periphery of debris sensor assembly 200 is axis symmetric.

Debris sensor assembly 200 further comprises a Hall-effect sensor 206 coupled to PCBA 208. In some embodiments, Hall-effect sensor 206 is configured to detect magnetic flux density within the periphery of debris sensor assembly 200 and any changes to the magnetic flux density caused by ferrous debris deposition along shaft 106. In some embodiments, axis-symmetry is maintained in the debris sensor assembly 200 to ensure the impact of debris collection is focused on Hall-effect sensor 206. As such, debris sensor assembly 200 may be configured for debris collection and sensing along the periphery of shaft 106 with uniformity and high sensitivity.

At least a portion of the one or more permanent magnets 202 comprises a cavity 214 in which a null field is established on a first or bottom end of the one or more permanent magnets 202. The null field of the cavity 214 may comprise a space where little to no magnetic flux exists. Hall-effect sensor 206 is placed inside the cavity 214 near the null field to reduce a magnetic field experienced by the Hall-effect sensor thereby avoiding saturation. As such, a Hall-effect sensor 206 coupled to a first side of PCBA 208 is positioned within the cavity 214 such that magnetic flux induced by the one or more permanent magnets 202 is lessened to prevent rendering the Hall-effect sensor 206 inoperable. In some embodiments the cavity 214 is centered such that the Hall-effect sensor 206 may be positioned along a central axis of the one or more permanent magnets 202. Hall-effect sensor 206 may be oriented at any angle (e.g., 0 to 360 degrees) within cavity 214 with respect to the axis of the one or more permanent magnets 202.

A second side of PCBA 208 may comprise a power source (e.g., wired or battery) and one or more electronic components configured to generate digital (e.g., Serial Peripheral Interface (SPI), Single Edge Nibble Transmission (SENT), Inter-Integrated Circuit (I2C), etc.) or analog (voltage, current, pulse-width modulation (PWM), etc.) signal output representation of measurement data based on readings from Hall-effect sensor 206. PCBA 208 may further comprise a microcontroller or computing entity and a communication interface for transmitting the digital or analog signal output to a monitoring computing system via digital (e.g., Controller Area Network (CAN)), analog signals, or via one or more wired or wireless communication protocols.

Debris collection and sensing may be effected by debris sensor assembly 200. In some embodiments, the debris sensor assembly 200 may be encapsulated and sealed within shaft 106 such that only shaft 106 is directly exposed to an operating environment. As such, no direct deposition of debris occurs on the debris sensor assembly 200. For example, when used in an environment comprising a fluid, no direct contact may be made between the fluid and the debris sensor assembly 200. Shaft 106 may comprise a non-ferrous housing that allows magnetic flux from the one or more permanent magnets 202 to pass through shaft 106 unimpeded and/or unaltered.

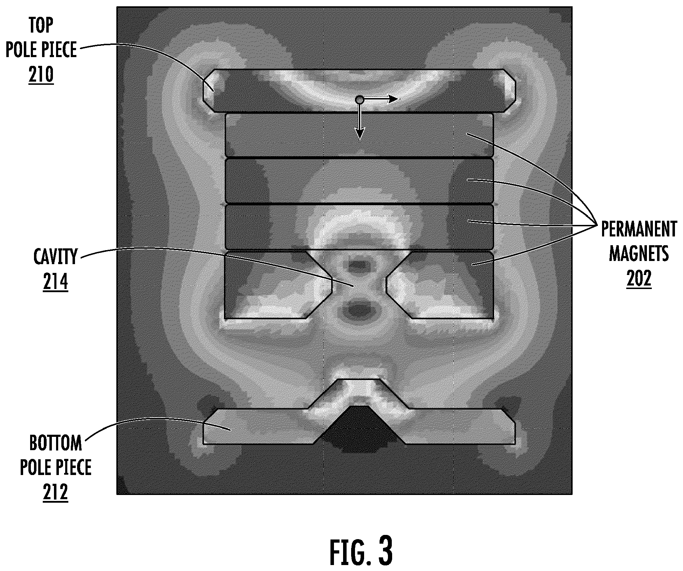

Debris sensor assembly 200 further comprises top pole piece 210 and bottom pole piece 214 which may be used to shape the magnetic flux from the one or more permanent magnets 202. The one or more permanent magnets 202, non-magnetic spacers 204, Hall-effect sensor 206, and PCBA 208 are configured between the top pole piece 210 and the bottom pole piece 212 within the shaft 106. According to various embodiments of the present disclosure, top pole piece 210 and the bottom pole piece 212 may comprise high permeable steel plates that act as flux concentrators to concentrate magnetic flux (from one or more permanent magnets 202) through Hall-effect sensor as well as through a length of the periphery of shaft 106, as depicted in FIG. 3. For example, the top pole piece 210 and the bottom pole piece 212 may focus or restrict the magnetic field induced by one or more permanent magnets 202 along a 360-degree periphery around debris sensor assembly 200.

The top pole piece 210 is coupled to or configured adjacently to (either in contact with or not in contact with) the one or more permanent magnets 202 at a second or top end that is opposite of the first or bottom end comprising the cavity 214. The bottom pole piece 212 is configured adjacent to a second side (opposite of Hall-effect sensor 206) of PCBA 208. The bottom pole piece 212, being closest to Hall-effect sensor 206 (than that of top pole piece 210) may be profiled (e.g., funnel-shaped towards the Hall-effect sensor 206) in such a way that augments magnetic flux (e.g., due to debris deposition) to be uniformly experienced by and concentrated on the Hall-effect sensor 206. In some embodiments, a gap exists between the bottom pole piece 212 and the second side of PCBA 208. The gap between the bottom pole piece 212 and the Hall-effect sensor 206 may be varied to change magnetic flux patterns as desired.

The capacity of debris sensor assembly 200 to collect and sense debris may be adjusted by adding or adjusting the one or more permanent magnets 202, the non-magnetic spacers 204, and/or adjusting top pole piece 210 and bottom pole piece 212 along the length of shaft 106. For example, areas of debris deposition on shaft 106 may be varied based on the length of debris sensor assembly 200 and its components, such as by varying the length or amount of one or more permanent magnets 202. As another example, uniformity of debris deposition along shaft 106 may be varied by varying thickness of the non-magnetic spacers 204 between magnets as well as changing gap between one or more permanent magnets 202 and top pole piece 210 and/or bottom pole piece 212. In yet another embodiment, areas of debris deposition on shaft 106 and detection of debris in the areas of debris deposition may be modified by varying shapes, configurations, and/or orientation of the one or more permanent magnets 202, the non-magnetic spacers 204, the top pole piece 210, or the bottom pole piece 212.

Various performance parameters of debris sensor assembly 200 associated with magnetic flux density detected by Hall-effect sensor 206 may be optimized by modifying the one or more permanent magnets 202, the non-magnetic spacers 204, the top pole piece 210, or the bottom pole piece 212. For example, a magnetic field experienced by Hall-effect sensor 206 may be configured to ensure that the Hall-effect sensor 206 is not saturated by modifying (i) size of the non-magnetic spacers 204 or gaps between the one or more permanent magnets 202, (ii) a gap between the one or more permanent magnets 202 and the top pole piece 210 and/or the bottom pole piece 212, (iii) a quantity or size of the one or more permanent magnets 202, or (iv) a shape/profile of the top pole piece 210 and/or the bottom pole piece 212.

According to various embodiments of the present disclosure, magnetic flux density experienced at the Hall-effect sensor 206 is scaled by varying the size of the non-magnetic spacers 204, gaps between the one or more permanent magnets 202, or gaps between the one or more permanent magnets 202 and the top pole piece 210 and/or the bottom pole piece 212, which thereby varies an area for debris deposition on shaft 106. Given that an deposition of ferrous debris along shaft 106 may create a low reluctance return path for a magnetic flux and increase the magnetic field at Hall-effect sensor 206 in proportion to an amount of the ferrous debris accumulated on the shaft 106, saturation of Hall-effect sensor 206 may be prevented by increasing the size of the non-magnetic spacers 204, gaps between the one or more permanent magnets 202, or gaps between the one or more permanent magnets 202 and the top pole piece 210 and/or the bottom pole piece 212, allowing for a greater range of debris deposition detection without exceeding a saturation limit of the Hall-effect sensor 206. Thus, by varying the size of the non-magnetic spacers 204, gaps between the one or more permanent magnets 202, or gaps between the one or more permanent magnets 202 and the top pole piece 210 and/or the bottom pole piece 212, an area for debris deposition on shaft 106 of a desired size may be configured. However, increasing the size of the non-magnetic spacers 204, gaps between the one or more permanent magnets 202, or gaps between the one or more permanent magnets 202 and the top pole piece 210 and/or the bottom pole piece 212 may come at a cost of lower detection resolution (e.g., large rate of change in magnetic flux density) and less detection linearity (e.g., consistency of measured magnetic flux density over various amounts of debris deposition). Similarly, in some other embodiments, smaller/fewer permanent magnets 202 causes a decrease in magnetic flux density experienced at the Hall-effect sensor 206 thereby preventing saturation and allowing for a greater range of debris deposition detection, however, at the cost of lower detection resolution and less detection linearity. Accordingly, based on a given application, a debris sensor assembly 200 may be designed based on a desired tradeoff between debris deposition amounts measurable and detection quality (e.g., detection resolution and/or detection linearity).

In some example embodiments, debris sensor plug 100 is used in an operating environment comprising ferrous debris (e.g., cause by wear or damage to one or more serviceable components) suspended or migrating in a fluid. The ferrous debris may be attracted to a magnetic field generated by the debris sensor assembly 200 causing the ferrous debris to accumulate along shaft 106. A deposition of the ferrous debris along shaft 106 may complete a magnetic circuit by creating a low reluctance return path for a magnetic flux and thereby increase the magnetic field at Hall-effect sensor 206 in proportion to an amount of ferrous debris accumulated on shaft 106. Changes in the amount of ferrous debris deposition on shaft 106 may cause corresponding changes in a magnetic flux density detected and captured by Hall-effect sensor 206. In some embodiments, raw data readings (e.g., in the form of analog or digital data readings) from Hall-effect sensor 206 may be converted into measurement readings comprising approximate values of magnetic flux density in a unit of measurement, such as gauss or tesla, or approximate values of debris deposition mass or weight, such as grams or ounces.

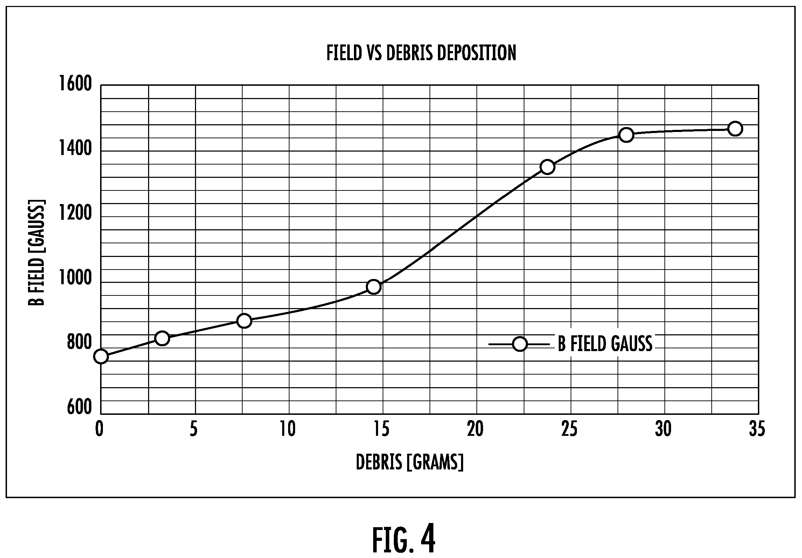

FIG. 4 depicts an example relationship between magnetic flux density and debris deposition on a debris sensor plug in accordance with various embodiments of the present disclosure. A Hall-effect sensor response may be configured against debris mass/weight using a linearization algorithm or lookup table such that real-time debris deposition may be monitored to avoid catastrophic failures in a monitored environment. In some example embodiments, for normal operation, an acceptable quantity of debris deposited may be within around 3-6 grams over a particular maintenance cycle. In some embodiments, measurement data from the Hall-effect sensor 206 may be used to determine debris deposition with respect to rate of deposition of debris. As such, based on a rate of change detected by the Hall-effect sensor 206, a sudden surge in quantity of debris may be detected in case of any catastrophic failure. In some embodiments, particle size of debris is distinguished based on readings from the Hall-effect sensor 206 thereby identifying specific component leading to failure as different components may have different permeability.

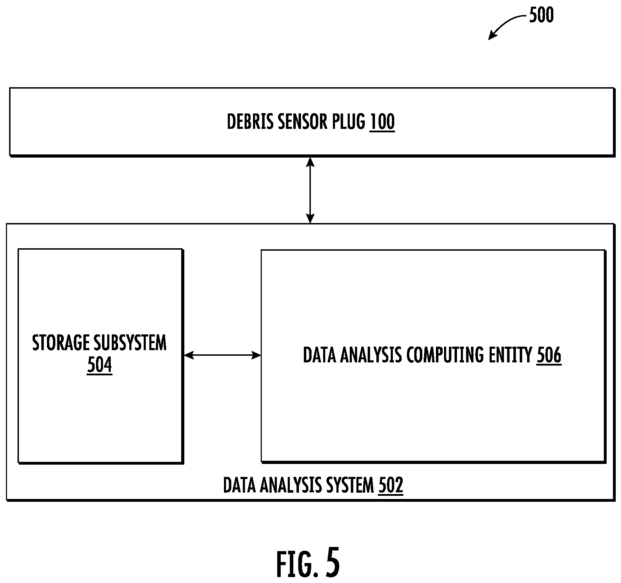

FIG. 5 provides an example overview of an architecture 500 in accordance with some embodiments of the present disclosure. The architecture 500 includes a data analysis system 502 configured to receive measurement data from debris sensor plug 100, monitor and/or process the measurement data, generate predictions of wear or failure based on the measurement data, and automatically initiate performance of one or more actions based on the predictions. The example architecture 500 may be used in a plurality of domains, such as industrial or manufacturing, and not limited to any specific application as disclosed herewith.

An example of an action that may be performed using the data analysis system 502 comprises receiving measurement data values from debris sensor plug 100 and determining an amount of debris deposition associated with the debris sensor plug 100, performing a diagnostic or analysis based on the determined amount of debris deposition, and displaying results of the diagnostic or analysis on a user interface. Other examples of actions that may be performed comprise generating a diagnostic report, displaying/providing resources, generating, and/or executing action scripts, generating alerts or reminders (e.g., maintenance or replacement of serviceable components associated with debris sensor plug 100 or debris sensor plug 100 itself) or generating one or more electronic communications based on the diagnostic or analysis.

In some embodiments, data analysis system 502 may communicate with a debris sensor plug 100 using one or more communication networks. Examples of communication networks include any wired or wireless communication network including, for example, a wired or wireless local area network (LAN), personal area network (PAN), metropolitan area network (MAN), wide area network (WAN), or the like, as well as any hardware, software, and/or firmware required to implement it (such as, e.g., network routers, and/or the like).

The data analysis system 502 may include a data analysis computing entity 506 and a storage subsystem 508. The data analysis computing entity 506 may be configured to receive measurement data from debris sensor plug 100, monitor and/or process the measurement data, generate predictions of wear or failure based on the measurement data, and automatically initiate performance of one or more actions based on the predictions. The storage subsystem 508 may be configured to store input data used by the data analysis computing entity 506 to perform various data analysis tasks. The storage subsystem 508 may include one or more storage units, such as multiple distributed storage units that are connected through a computer network. Each storage unit in the storage subsystem 508 may store at least one of one or more data assets and/or one or more data about the computed properties of one or more data assets. Moreover, each storage unit in the storage subsystem 508 may include one or more non-volatile storage or memory media including, but not limited to, hard disks, ROM, PROM, EPROM, EEPROM, flash memory, MMCs, SD memory cards, Memory Sticks, CBRAM, PRAM, FeRAM, NVRAM, MRAM, RRAM, SONOS, FJG RAM, Millipede memory, racetrack memory, and/or the like.

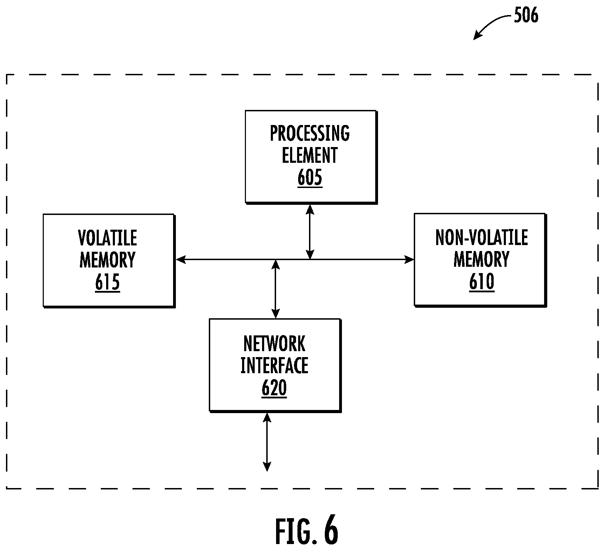

FIG. 6 provides an example data analysis computing entity 506 in accordance with some embodiments of the present disclosure. In general, the terms computing entity, computer, entity, device, system, and/or similar words used herein interchangeably may refer to, for example, one or more computers, computing entities, desktops, mobile phones, tablets, phablets, notebooks, laptops, distributed systems, kiosks, input terminals, servers or server networks, blades, gateways, switches, processing devices, processing entities, set-top boxes, relays, routers, network access points, base stations, the like, and/or any combination of devices or entities adapted to perform the functions, operations, and/or processes described herein. Such functions, operations, and/or processes may include, for example, transmitting, receiving, operating on, processing, displaying, storing, determining, creating/generating, monitoring, evaluating, comparing, and/or similar terms used herein interchangeably. In some embodiments, these functions, operations, and/or processes may be performed on data, content, information, and/or similar terms used herein interchangeably.

As shown in FIG. 6, in some embodiments, the data analysis computing entity 506 may include, or be in communication with, one or more processing elements 605 (also referred to as processors, processing circuitry, and/or similar terms used herein interchangeably) that communicate with other elements within the data analysis computing entity 606 via a bus, for example. As will be understood, the processing element 605 may be embodied in a number of different ways.

For example, the processing element 605 may be embodied as one or more complex programmable logic devices (CPLDs), microprocessors, multi-core processors, coprocessing entities, application-specific instruction-set processors (ASIPs), microcontrollers, and/or controllers. Further, the processing element 605 may be embodied as one or more other processing devices or circuitry. The term circuitry may refer to an entirely hardware embodiment or a combination of hardware and computer program products. Thus, the processing element 605 may be embodied as integrated circuits, application specific integrated circuits (ASICs), field programmable gate arrays (FPGAs), programmable logic arrays (PLAs), hardware accelerators, other circuitry, and/or the like.

As will therefore be understood, the processing element 605 may be configured for a particular use or configured to execute instructions stored in volatile or non-volatile media or otherwise accessible to the processing element 605. As such, whether configured by hardware or computer program products, or by a combination thereof, the processing element 605 may be capable of performing steps or operations according to embodiments of the present disclosure when configured accordingly.

In some embodiments, the data analysis computing entity 506 may further include, or be in communication with, non-volatile media (also referred to as non-volatile storage, memory, memory storage, memory circuitry, and/or similar terms used herein interchangeably). In some embodiments, the non-volatile storage or memory may include one or more non-volatile memory 610, including, but not limited to, hard disks, ROM, PROM, EPROM, EEPROM, flash memory, MMCs, SD memory cards, Memory Sticks, CBRAM, PRAM, FeRAM, NVRAM, MRAM, RRAM, SONOS, FJG RAM, Millipede memory, racetrack memory, and/or the like.

As will be recognized, the non-volatile storage or memory media may store databases, database instances, database management systems, data, applications, programs, program modules, scripts, source code, object code, byte code, compiled code, interpreted code, machine code, executable instructions, and/or the like. The term database, database instance, database management system, and/or similar terms used herein interchangeably may refer to a collection of records or data that is stored in a computer-readable storage medium using one or more database models, such as a hierarchical database model, network model, relational model, entityrelationship model, object model, document model, semantic model, graph model, and/or the like.

In some embodiments, the data analysis computing entity 506 may further include, or be in communication with, volatile media (also referred to as volatile storage, memory, memory storage, memory circuitry, and/or similar terms used herein interchangeably). In some embodiments, the volatile storage or memory may also include one or more volatile memory 615, including, but not limited to, RAM, DRAM, SRAM, FPM DRAM, EDO DRAM, SDRAM, DDR SDRAM, DDR2 SDRAM, DDR3 SDRAM, RDRAM, TTRAM, T-RAM, Z-RAM, RIMM, DIMM, SIMM, VRAM, cache memory, register memory, and/or the like.

As will be recognized, the volatile storage or memory media may be used to store at least portions of the databases, database instances, database management systems, data, applications, programs, program modules, scripts, source code, object code, byte code, compiled code, interpreted code, machine code, executable instructions, and/or the like being executed by, for example, the processing element 605. Thus, the databases, database instances, database management systems, data, applications, programs, program modules, scripts, source code, object code, byte code, compiled code, interpreted code, machine code, executable instructions, and/or the like may be used to control certain aspects of the operation of the data analysis computing entity 506 with the assistance of the processing element 605 and operating system.

As indicated, in some embodiments, the data analysis computing entity 506 may also include one or more network interfaces 620 for communicating with debris sensor plug 100 and various computing entities, such as by communicating data, content, information, and/or similar terms used herein interchangeably that may be transmitted, received, operated on, processed, displayed, stored, and/or the like. Such communication may be executed using a wired data transmission protocol, such as fiber distributed data interface (FDDI), digital subscriber line (DSL), Ethernet, asynchronous transfer mode (ATM), frame relay, data over cable service interface specification (DOCSIS), or any other wired transmission protocol. Similarly, the data analysis computing entity 506 may be configured to communicate via wireless external communication networks using any of a variety of protocols, such as general packet radio service (GPRS), Universal Mobile Telecommunications System (UMTS), Code Division Multiple Access 2000 (CDMA2000), CDMA2000 1X (1xRTT), Wideband Code Division Multiple Access (WCDMA), Global System for Mobile Communications (GSM), Enhanced Data rates for GSM Evolution (EDGE), Time Division-Synchronous Code Division Multiple Access (TD-SCDMA), Long Term Evolution (LTE), Evolved Universal Terrestrial Radio Access Network (E-UTRAN), Evolution-Data Optimized (EVDO), High Speed Packet Access (HSPA), High-Speed Downlink Packet Access (HSDPA), IEEE 802.11 (Wi-Fi), Wi-Fi Direct, 802.16 (WiMAX), ultra-wideband (UWB), infrared (IR) protocols, near field communication (NFC) protocols, Wibree, Bluetooth protocols, wireless universal serial bus (USB) protocols, and/or any other wireless protocol.

Although not shown, the data analysis computing entity 506 may include, or be in communication with, one or more input elements, such as a keyboard input, a mouse input, a touch screen/display input, motion input, movement input, audio input, pointing device input, joystick input, keypad input, and/or the like. The data analysis computing entity 506 may also include, or be in communication with, one or more output elements (not shown), such as audio output, video output, screen/display output, motion output, movement output, and/or the like.

As disclosed herewith, debris sensor plug 100 may be used in conjunction with data analysis system 502 to monitor an operating environment, such as machinery or equipment, and determine when to employ preventative maintenance to avoid costly damage to system/components and/or dealing with an unscheduled failure leading to safety concerns, downtime / lost time cost, lost production, and unnecessary overtime, as well as to avoid prematurely performing maintenance on or replacing equipment when not needed.

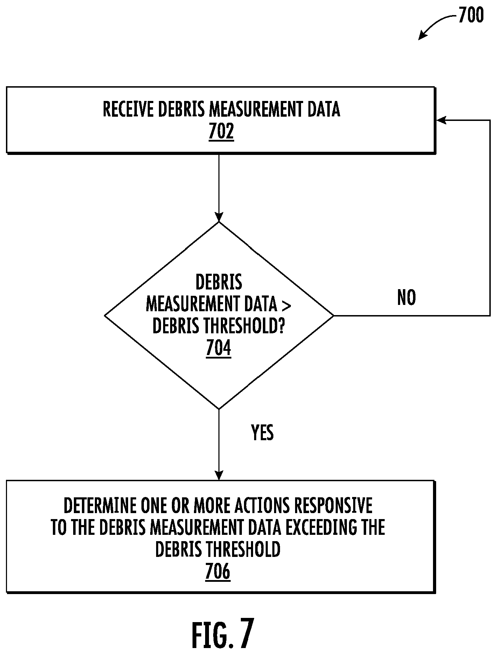

Referring now to FIG. 7, an example flow diagram illustrating an example method for monitoring debris deposition in an operating environment in accordance with some example embodiments of the present disclosure.

It is noted that each block of a flowchart, and combinations of blocks in the flowchart, may be implemented by various means such as hardware, firmware, circuitry, and/or other devices associated with execution of software including one or more computer program instructions. For example, one or more of the steps/operations described in FIG. 7 may be embodied by computer program instructions, which may be stored by a non-transitory memory of an apparatus employing an embodiment of the present disclosure and executed by a processor component in an apparatus (such as, but not limited to, data analysis computing entity 506). For example, these computer program instructions may direct the processor component to function in a particular manner, such that the instructions stored in the computer-readable storage memory produce an article of manufacture, the execution of which implements the function specified in the flowchart block(s).

In some embodiments, at step/operation 702, the data analysis computing entity 506 receives debris measurement data from debris sensor plug 100. The debris measurement data may comprise raw data in the form of analog or digital signals generated by Hall-effect sensor 206 based on magnetic flux induced on the Hall-effect sensor 206 as a result of debris deposition (or lack thereof).

In some embodiments, subsequent to step/operation 702, the example method proceeds to step/operation 704, where the data analysis computing entity 506 processes, monitors, and/or analyzes the debris measurement data and determines whether the debris measurement data exceeds a debris threshold. The measurement data may be associated with magnetic flux density values detected by Hall-effect sensor 206. The magnetic flux density values may be representative of an amount of ferrous debris deposition on debris sensor plug 100. In some embodiments, determining whether the debris measurement data exceeds a debris threshold further comprises using the measurement data to determine an approximate mass/weight of debris deposition on debris sensor plug 100 based on a linearization algorithm or lookup table. The approximate mass/weight of debris deposition may be compared with a debris threshold comprising a maximum total deposition.

In some embodiments, a rate of change associated with debris deposition is determined based on the measurement data and previous measurement data. The rate of change may be compared with a debris threshold comprising a maximum rate of change associated with debris deposition. In some other embodiments, determining whether the debris measurement data exceeds a debris threshold further comprises determining particle size of debris deposition based on the measurement data and comparing the determined particle size with a debris threshold comprising a particle size threshold. As an example, a particle size threshold may be associated with debris from a specific component, and thus, allowing data analysis computing entity 506 to determine wear or failure of a specific component.

If the debris measurement data is not greater than the debris threshold, the data analysis computing entity 506 continues to receive and monitor the debris measurement data. In some embodiments, subsequent to step/operation 704, if the debris measurement data is greater than the debris threshold, the example method proceeds to step/operation 706, where the data analysis computing entity 506 determines one or more actions responsive to the debris measurement data exceeding the debris threshold. In some embodiments, determining the one or more actions comprises generating a prediction of wear or failure based on the debris threshold exceeded. The prediction of wear or failure may be used to determine appropriate one or more actions to respond to the debris measurement data exceeding the debris threshold. For example, distinguishing regular wear from system breakdown may determine actions, such as routine maintenance, replacement, or repairs, which may be performed to avoid failures and/or unnecessary downtime.

It is to be understood that the disclosure is not to be limited to the specific embodiments disclosed, and that modifications and other embodiments are intended to be included within the scope of the appended claims. Although specific terms are employed herein, they are used in a generic and descriptive sense only and not for purposes of limitation, unless described otherwise.

## Claims

1. A debris sensor apparatus comprising:
a head portion (102);
a male-threaded portion (104); and
a shaft (106) comprising a debris sensor assembly (200), the debris sensor assembly (200) comprising:
one or more permanent magnets (202) comprising a cavity (214) on a first end;
a Hall-effect sensor (206) configured within the cavity (214);
a first pole piece (210) configured adjacent to a second end of the one or more permanent magnets (202); and
a second pole piece (212) configured adjacent to the first end,
**characterised in that**
a null magnetic field space is established within the cavity (214), and the Hall-effect sensor is positioned within the null magnetic field space.

2. The debris sensor apparatus of claim 1 further comprising a printed circuit board assembly (208) coupled to the Hall-effect sensor.

3. The debris sensor apparatus of claim 2, wherein the printed circuit board (208) comprises one or more processors and a communication interface configured to transmit signal output associated with the Hall-effect sensor to a data analysis computing entity.

4. The debris sensor apparatus of claim 3, wherein the signal output is representative of debris measurement data comprising one or more values of magnetic flux density.

5. The debris sensor apparatus of claim 4, wherein the data analysis computing entity is configured to generate one or more actions responsive to the debris measurement data exceeding a debris threshold.

6. The debris sensor apparatus of claim 5 wherein the debris threshold is associated with a maximum rate of change associated with debris deposition on the shaft (106) or a maximum amount of debris deposition on the shaft (106).

7. The debris sensor apparatus of claim 1, wherein the Hall-effect sensor is configured to detect magnetic flux density.

8. The debris sensor apparatus of claim 7, wherein the Hall-effect sensor is configured to detect an increase to the magnetic flux density in proportion to an amount of ferrous debris deposition on the shaft (106).

9. The debris sensor apparatus of claim 1, wherein the one or more permanent magnets (202) comprise an array of a plurality of magnet elements and one or more non-magnetic spacers (204) configured therebetween the plurality of magnet elements.

10. The debris sensor apparatus of claim 1, wherein the Hall-effect sensor is configured along a central axis of the one or more permanent magnets (202).

11. The debris sensor apparatus of claim 1, wherein the top piece and the bottom pole piece comprise flux concentrators that concentrate magnetic flux associated with the one or more permanent magnets (202) through the Hall-effect sensor and through a length of a periphery of shaft (106).

12. The debris sensor apparatus of claim 1, wherein the shaft (106) comprises a non-ferrous housing.

13. The debris sensor apparatus of claim 1, wherein the Hall-effect sensor is oriented in the cavity (214) at a given angle with respect to an axis of the one or more permanent magnets (202).

14. The debris sensor apparatus of claim 1, wherein the second pole piece comprises a funnel shape towards the Hall-effect sensor.

15. The debris sensor apparatus of claim 1, wherein the debris sensor assembly (200) further comprises a gap between the bottom pole piece and the Hall-effect sensor.

## Patentansprüche

1. Partikelsensoreinrichtung, umfassend:
einen Kopfteil (102);
einen Außengewindeteil (104); und
eine Welle (106), umfassend eine Partikelsensorbaugruppe (200), die Partikelsensorbaugruppe (200) umfassend:
einen oder mehrere Permanentmagneten (202), umfassend einen Hohlraum (214) an einem ersten Ende;
einen Hall-Effekt-Sensor (206), der innerhalb des Hohlraums (214) konfiguriert ist;
ein erstes Polstück (210), das benachbart zu einem zweiten Ende des einen oder der mehreren Permanentmagneten (202) konfiguriert ist; und
ein zweites Polstück (212), das benachbart zu dem ersten Ende konfiguriert ist,
**gekennzeichnet dadurch, dass**
ein magnetischer Nullfeldraum innerhalb des Hohlraums (214) hergestellt wird und der Hall-Effekt-Sensor innerhalb des magnetischen Nullfeldraums positioniert ist.

2. Partikelsensoreinrichtung nach Anspruch 1, ferner umfassend eine Leiterplattenbaugruppe (208), die mit dem Hall-Effekt-Sensor gekoppelt ist.

3. Partikelsensoreinrichtung nach Anspruch 2, wobei die Leiterplatte (208) einen oder mehrere Prozessoren und eine Kommunikationsschnittstelle umfasst, die dazu konfiguriert ist, eine dem Hall-Effekt-Sensor zugehörige Signalausgabe an eine Datenanalyserecheneinheit zu übertragen.

4. Partikelsensoreinrichtung nach Anspruch 3, wobei die Signalausgabe repräsentativ für Partikelmessungsdaten ist, umfassend einen oder mehrere Werte einer Magnetflussdichte.

5. Partikelsensoreinrichtung nach Anspruch 4, wobei die Datenanalyserecheneinheit dazu konfiguriert ist, eine oder mehrere Maßnahmen als Antwort darauf zu erzeugen, dass die Partikelmessungsdaten einen Partikelschwellenwert überschreiten.

6. Partikelsensoreinrichtung nach Anspruch 5, wobei der Partikelschwellenwert einer maximalen Änderungsrate, die einer Partikelablagerung auf der Welle (106) zugehörig ist, oder einer maximalen Menge von Partikelablagerung auf der Welle (106) zugehörig ist.

7. Partikelsensoreinrichtung nach Anspruch 1, wobei der Hall-Effekt-Sensor dazu konfiguriert ist, eine Magnetflussdichte zu detektieren.

8. Partikelsensoreinrichtung nach Anspruch 7, wobei der Hall-Effekt-Sensor dazu konfiguriert ist, eine Zunahme der Magnetflussdichte proportional zu einer Menge von Eisenpartikelablagerung auf der Welle (106) zu detektieren.

9. Partikelsensoreinrichtung nach Anspruch 1, wobei der eine oder die mehreren Permanentmagneten (202) eine Anordnung einer Vielzahl von Magnetelementen und einen oder mehrere nicht-magnetische Abstandshalter (204), die zwischen der Vielzahl von Magnetelementen konfiguriert sind, umfassen.

10. Partikelsensoreinrichtung nach Anspruch 1, wobei der Hall-Effekt-Sensor entlang einer Mittelachse des einen oder der mehreren Permanentmagneten (202) konfiguriert ist.

11. Partikelsensoreinrichtung nach Anspruch 1, wobei das obere Stück und das untere Polstück Flusskonzentratoren umfassen, die einen Magnetfluss, der dem einen oder den mehreren Permanentmagneten (202) zugehörig ist, durch den Hall-Effekt-Sensor und durch eine Länge eines Umfangs der Welle (106) konzentrieren.

12. Partikelsensoreinrichtung nach Anspruch 1, wobei die Welle (106) ein Nichteisen-Gehäuse umfasst.

13. Partikelsensoreinrichtung nach Anspruch 1, wobei der Hall-Effekt-Sensor in dem Hohlraum (214) in einem vorgegebenen Winkel in Bezug auf eine Achse des einen oder der mehreren Permanentmagneten (202) ausgerichtet ist.

14. Partikelsensoreinrichtung nach Anspruch 1, wobei das zweite Polstück eine Trichterform zu dem Hall-Effekt-Sensor hin umfasst.

15. Partikelsensoreinrichtung nach Anspruch 1, wobei die Partikelsensorbaugruppe (200) ferner einen Spalt zwischen dem unteren Polstück und dem Hall-Effekt-Sensor umfasst.

## Revendications

1. Appareil capteur de débris comprenant :
une partie de tête (102) ;
une partie à filetage mâle (104) ; et
un arbre (106) comprenant un ensemble capteur de débris (200), l'ensemble capteur de débris (200) comprenant :
un ou plusieurs aimants permanents (202) comprenant une cavité (214) sur une première extrémité ;
un capteur à effet Hall (206) configuré à l'intérieur de la cavité (214) ;
une première pièce polaire (210) configurée adjacente à une deuxième extrémité du ou des aimants permanents (202) ; et
une deuxième pièce polaire (212) configurée adjacente à la première extrémité,
**caractérisé en ce que**
un espace à champ magnétique nul est établi à l'intérieur de la cavité (214), et le capteur à effet Hall est positionné à l'intérieur de l'espace à champ magnétique nul.

2. Appareil capteur de débris selon la revendication 1, comprenant en outre un ensemble carte de circuit imprimé (208) couplé au capteur à effet Hall.

3. Appareil capteur de débris selon la revendication 2, dans lequel la carte de circuit imprimé (208) comprend un ou plusieurs processeurs et une interface de communication configurée pour transmettre une sortie de signal associée au capteur à effet Hall à une entité informatique d'analyse de données.

4. Appareil capteur de débris selon la revendication 3, dans lequel la sortie de signal est représentative des données de mesure de débris comprenant une ou plusieurs valeurs de densité de flux magnétique.

5. Appareil capteur de débris selon la revendication 4, dans lequel l'entité informatique d'analyse de données est configurée pour générer une ou plusieurs actions en réponse lorsque les données de mesure de débris dépassent un seuil de débris.

6. Appareil capteur de débris selon la revendication 5, dans lequel le seuil de débris est associé à un taux maximal de changement associé à un dépôt de débris sur l'arbre (106) ou à une quantité maximale de dépôt de débris sur l'arbre (106).

7. Appareil capteur de débris selon la revendication 1, dans lequel le capteur à effet Hall est configuré pour détecter une densité de flux magnétique.

8. Appareil capteur de débris selon la revendication 7, dans lequel le capteur à effet Hall est configuré pour détecter une augmentation de la densité de flux magnétique en proportion à une quantité de dépôt de débris ferreux sur l'arbre (106).

9. Appareil capteur de débris selon la revendication 1, dans lequel le ou les aimants permanents (202) comprennent un réseau d'une pluralité d'éléments d'aimants et un ou plusieurs espaceurs non magnétiques (204) configurés entre la pluralité d'éléments d'aimants.

10. Appareil capteur de débris selon la revendication 1, dans lequel le capteur à effet Hall est configuré le long d'un axe central du ou des aimants permanents (202).

11. Appareil capteur de débris selon la revendication 1, dans lequel la pièce supérieure et la pièce polaire inférieure comprennent des concentrateurs de flux qui concentrent un flux magnétique associé à ou aux aimants permanents (202) à travers le capteur à effet Hall et à travers une longueur d'une périphérie de l'arbre (106).

12. Appareil capteur de débris selon la revendication 1, dans lequel l'arbre (106) comprend un corps non ferreux.

13. Appareil capteur de débris selon la revendication 1, dans lequel le capteur à effet Hall est orienté dans la cavité (214) à un angle donné par rapport à un axe du ou des aimants permanents (202).

14. Appareil capteur de débris selon la revendication 1, dans lequel la deuxième pièce polaire comprend une forme d'entonnoir vers le capteur à effet Hall.

15. Appareil capteur de débris selon la revendication 1, dans lequel l'ensemble capteur de débris (200) comprend en outre un intervalle entre la pièce polaire inférieure et le capteur à effet Hall.
